# EUROPÄISCHE PATENTANMELDUNG

(11) **EP 1 602 364 A1**
(43) Veröffentlichungstag der Anmeldung: **07.12.2005**
(21) Anmeldenummer: 04012286.3
(22) Anmeldetag: 25.05.2004
(51) Int. Cl.: A61K 9/50, A61K 35/78

(54) **Mikrokapseln (XXX), enthaltend Pflanzenextrakte, und Verfahren zu deren Herstellung**

(71) Anmelder: Cognis IP Management GmbH, 40589 Düsseldorf (DE)
(72) Erfinder: Viladot Petit, Joseph-Lluis, Dr., 08018 Barcelona (ES); Asensio, Juan-Antonio, Dr., 08820 El Prat de Llobregat(Barcelona) (ES); Rull Prous, Santiago, 08034 Barcelona (ES); Escudero, Joaquin, 08015 Barcelona (ES); Blasquez, Jose Fernandez, 08228 Terrassa (ES)

(57) **Zusammenfassung**

Vorgeschlagen werden Mikrokapseln mit mittleren Durchmessern im Bereich von 0,0001 bis 5 mm, die dadurch erhältlich sind , dass man
(a) wässrige Zubereitungen von Polymeren, die erst bei pH-Werten oberhalb von 5,5 löslich sind auf einen alkalischen pH-Wert einstellt,
(b) die so erhaltenen Lösungen mit Pflanzenextrakten vermischt und
(c) die Zubereitungen einer Sprühtrocknung unterwirft.

## Beschreibung

### Gebiet der Erfindung

Die Erfindung befindet sich auf dem Gebiet der Nahrungsmittelergänzungsstoffe und betrifft neue, mit Pflanzenextrakten beladene Mikrokapseln für die orale Aufnahme, die im sauren Bereich beständig sind und sich erst unter den alkalischen Bedingungen nach Passieren des sauren Milieus des Magen auflösen und den Wirkstoff freisetzen.

### Stand der Technik

Pflanzenextrakte enthalten eine Vielzahl von physiologisch aktiven Wirkstoffen, wie beispielsweise Polyphenolen, die ein weites Spektrum von Eigenschaften aufweisen, das von der Bekämpfung von Arthritis, über die Linderung von Klimakteriumsbeschwerden bis hin zur Prophylaxe gegen Alzheimer oder Krebs reicht. Die Aufnahme dieser Wirkstoffe erfolgt vorzugsweise oral, wobei es erforderlich ist, die aktiven Prinzipien ohne Metabolisierung an den richtigen Ort zu befördern. Im Grundsatz kommt hierfür die Verkapselung der Pflanzenextrakte in einer stabilen Matrix in Betracht, das Problem hierbei besteht jedoch darin, dass die Kapseln des Stands der Technik säurelöslich oder allgemein pH-Wert resistent sind. Somit werden die einen Systeme bereits beim Passieren des Magens mit seinem sauren Milieu aufgelöst und die Wirkstoffe zu früh freigesetzt oder aber die Kapseln passieren den Magen unbeschadet, setzen aber auch unter den nachfolgend alkalischen Bedingungen die Wirkstoffe nicht frei.

Die Aufgabe der vorliegenden Erfindung hat somit darin bestanden, neue Mikrokapseln zur Verfügung zu stellen, die einen wirksamen Gehalt an Pflanzenextrakten aufweisen und dabei einerseits im alkalischen Bereich löslich und andererseits im sauren Bereich, speziell bei pH-Werten oberhalb von 5,5 löslich sind. Des weiteren muss die Auswahl des Verkapselungsmaterials so erfolgen, dass chemische Wechselwirkungen mit den aktiven Prinzipien der Wirkstoffe ausgeschlossen ist.

### Beschreibung der Erfindung

Gegenstand der Erfindung sind Mikrokapseln mit mittleren Durchmessern im Bereich von 0,0001 bis 5, vorzugsweise 0,05 bis 0,2 mm, die dadurch erhältlich sind , dass man
(a) wässrige Zubereitungen von Polymeren, die erst bei pH-Werten oberhalb von 5,5 löslich sind, auf einen alkalischen pH-Wert einstellt,
(b) die so erhaltenen Lösungen mit Pflanzenextrakten vermischt und
(c) die Zubereitungen einer Sprühtrocknung unterwirft.

Überraschenderweise wurde gefunden, dass sich durch Verkapselung von Pflanzenextrakten in speziellen Polymeren und nachfolgende Sprühtrockung Zubereitungen für die orale Aufnahme erhalten lassen, die den Wirkstoff erst nach Passieren des Magens am gewünschten Ort freisetzen. Die Polymeren haben des weiteren den Vorzug, dass sie sich gegenüber den pflanzlichen Wirkstoffen chemisch inert verhalten.

Ein weiterer Gegenstand der vorliegenden Erfindung betrifft ein Verfahren zur Herstellung von Mikrokapseln mit mittleren Durchmessern im Bereich von 0,0001 bis 5 mm, vorzugsweise 0,05 bis 0,2 mm, bei dem man
(a) wässrige Zubereitungen von Polymeren, die erst bei pH-Werten oberhalb von 5,5 löslich sind, auf einen alkalischen pH-Wert einstellt,
(b) die so erhaltenen Lösungen mit Pflanzenextrakten vermischt und
(c) die Zubereitungen einer Sprühtrocknung unterwirft.

### Polymere

Die Auswahl der Polymere richtet sich in erster Linie nach ihrer Löslichkeit bei unterschiedlichen pH-Werten. Konkret müssen die Polymere im alkalischen Bereich unlöslich, im sauren Milieu hingegen leicht löslich sein. Des weiteren müssen die Polymeren natürlich auch befähigt sein, bei der pH-induzierten Präzipitation Mikrokapseln zu bilden und die in der Lösung enthaltenen Wirkstoffe einzuschließen. Für diesen Zweck kommen neben Polyacrylaten, Polymethacrylaten, und Hydroxypropylmethylcellulose insbesondere Hydroxypropylmethylcellulosephthalat in Frage. Zur Herstellung der Mikrokapseln werden wässrige Zubereitungen der Polymeren hergestellt und durch Zugabe von wässriger Alkalihydroxidlösung auf einen pH-Wert im Bereich von 7,5 bis 11, vorzugsweise 8 bis 10 eingestellt.

### Pflanzenextrakte

Üblicherweise sind die Pflanzenextrakte, die im Sinne der vorliegenden Erfindung Verwendung finden, ausgewählt aus der Gruppe, die gebildet wird von *Ginkgo biloba, Oleacea europensis, Glyzyrrhiza glabra, Vaccinium myrtillus, Trifolium pratense, Litchi sinensis, Vitis vinifera, Brassica oleracea, Punica granatum, Petroselinium crispum, Centella asiatica, Passiflora incarnata, Medicago sativa, Valeriana officinalis, Castanea sativa, Salix alba* sowie *Hapagophytum procumbens.* Im folgenden wird kurz auf die Zusammensetzung und die wesentlichen aktiven Wirkstoffe in den Extrakten eingegangen.

### • Ginkgo biloba

Die aktiven Wirkstoffe der Extrakte, die aus den Blättern des Ginkgobaumes *(Ginkgo biloba)* gewonnen werden, sind Flavonoidglycosides, welche unter anderem (Iso)Quercitinglycoside, Kaempferol, Kaempferol-3-rhamnoside, Isorhamnetin, Luteolinglycoside, Sitosterolglycoside und insbesondere hexacyclische Terpenlactone, die sogenannten Ginkgolide A, B, C, J, M und Bilobalide enthalten.

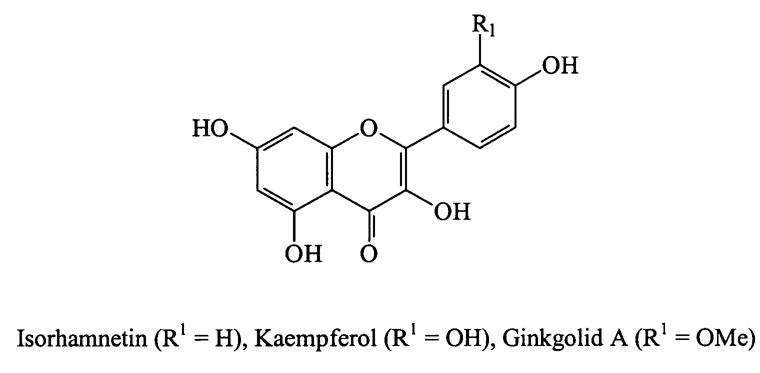

### • Oleacea europensis

Der Hauptbestandteil der Blätter des Olivenbaums *(Oleacea europensis)* ist das Antioxidants Oleuropein, das auch die wichtigste Quelle für Hydroxytyrosol darstellt.

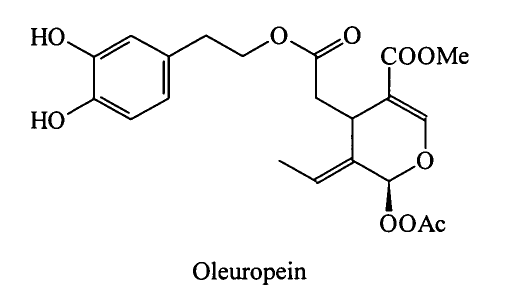

### • Glyzyrrhiza glabra

Hauptbestandteil des Extraktes der Süßwurzel *Glyzyrrhiza glabra* ist die Glyzyrrhetinsäure.

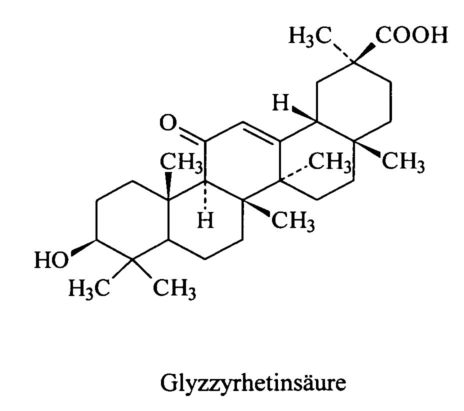

### • Vaccinium myrtillus

Extrakte der gemeinen Blaubeere *(Vaccinium myrtillus)* enthalten eine Mischung von wenigstens 15 verschiedenen Anthocyanosides, wie beispielsweise dem folgenden:

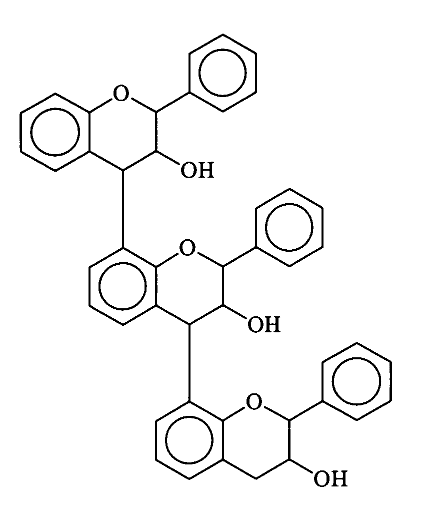

Üblicherweise, weisen die Extrakte 20 bis 25 Gew.-% Anthocyanoside, 5 bis 10 Gew.-% Tannine sowie geringe Mengen verschiedener Alkaloide, wie z.B. Myrtin und Epimyrtin, Phenolsäuren sowie Glycoside von Quercitrin, Isoquercitrin und Hyperosid auf.

### • Trifolium pratense

Die Hauptbestandteile der Extrakte des Rotklees (*Trifolium pratense*) sind Isoflavone, wie z.B. Daidzein, Genestein, Formononentin and Biochanin A sowie deren Glucosides wie z.B. Ononin oder Sissostrin:

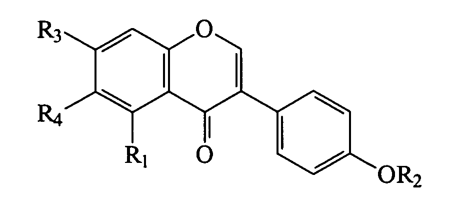

| Isoflavonglucoside | R₁ | R₂ | R₃ | R₄ |
|---|---|---|---|---|
| Daizidin | H | H | Glucose | H |
| Genistin | H | H | Glucose | OH |
| Ononin | H | CH₃ | Glucose | H |
| Sissostrin | H | CH₃ | Glucose | OH |

### • Litchi sinensis

Extrakte, die aus den Schalen der Litchifrucht (*Litchi sinensis*) gewonnen werden, weisen hohe Gehalte an Flavonerivaten auf, wie z.B. 2-Phenyl-4H-1-benzopyranen, Flavanen, Flavan-3-olen (Catechinen, Catechinoligomeren), Flavan-3,4-diolen (Leucoanthocyaniden), Flavonen, Flavonolen und Flavononen. Der Hauptbestandteil wird jedoch ausgemacht von kondensierten Tanninen, sogenannten Procyanodolen (OPC). Diese Stoffe enthalten 2 bis 8 Monomere des Catechins oder eines Catechintyps, wie z.B. Procyanidin, Proanthocynidin, Procyanidole, Oligoprocyanidin, Leucoanthocyanidin, Leucodelphinin, Leucocyanin and Anthocyanogen. OPC, vorzugsweise Proanthocyanidin A2 (OPC A2) verhalten sich wie Vitamin P, vor allem mit Hinblick auf die Inhibierung von Matrixmetallproteinasen.

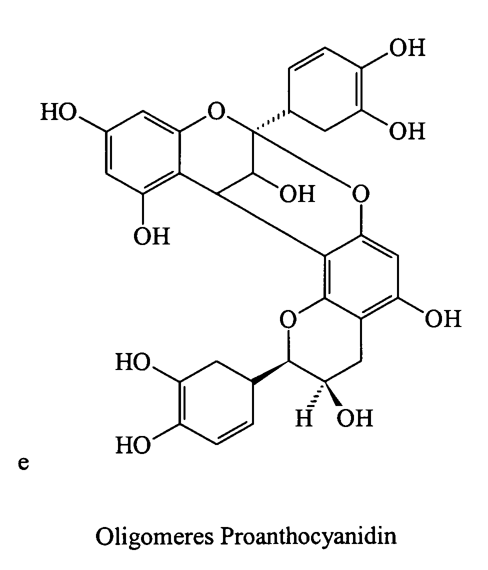

### • Vitis vinifera

Die Hauptbestandteile Extrakte aus Blättern, Wurzeln und insbesondere Schalen der Weintraube (*Vitis vinifera*) sind Polyphenole vom oben beschriebenen OPC-Typ.

### • Brassica oleracea

Die Hauptbestandteile der Extrakte des Blumenkohls (*Brassica oleracea*) sind Aminosäuren, insbesondere Methionin und Cystein sowie die Glucosinolate, wie z.B. Glucoraphanin.

### • Punica granatum

In den Extrakten des Granatapfels (*Punica granatum*) finden sich neben Zuckern und Zitronensäure insbesondere Delphinidin-1,2-glykoside sowie deren Aglykone.

### • Petroselinium crispum

Hauptbestandteil des fetten Öls der Petersilie (*Petroselinium crispum*) ist die Petroselinsäure. Die Extrakte hingegen zeigen hohe Gehalte an Apiol (1-Allyl-2,5-dimethoxy-3,4-(methylendioxy)benzol,), sowie Apiin, Myristicin, Pinen und Selinen.

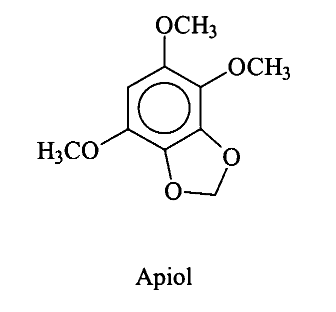

### • Centella asiatica

Hauptbestandteile der Extrakte der *Centella asiatica* sind hochkondensierte Naphthensäuren, speziell Asiaticasäure, Madecassicasäure sowie deren Glycoside.

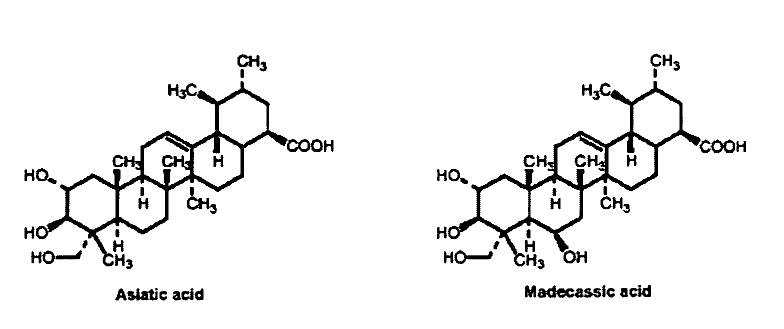

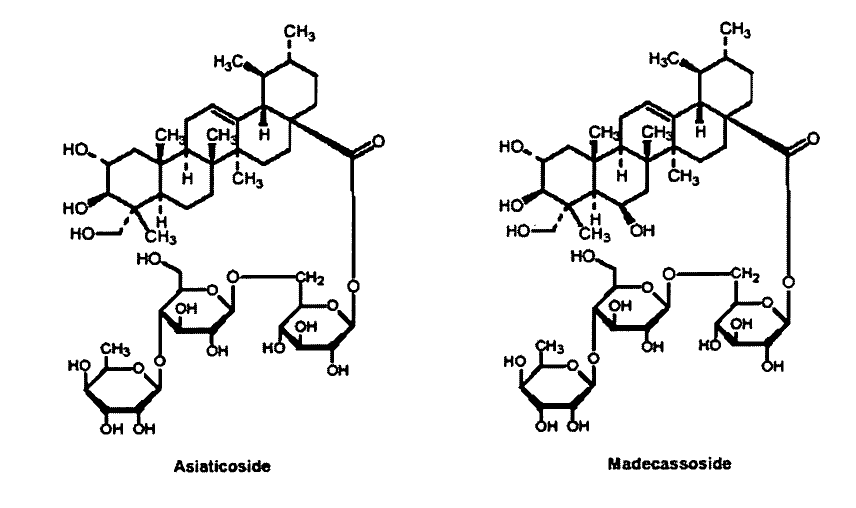

### • Passiflora incarnata

Extrakte der Passionsfrucht (*Passiflora incarnata*) sind reich an Flavonen vom Typ des Apigenins und Luteolins sowie deren C-Glycoside.

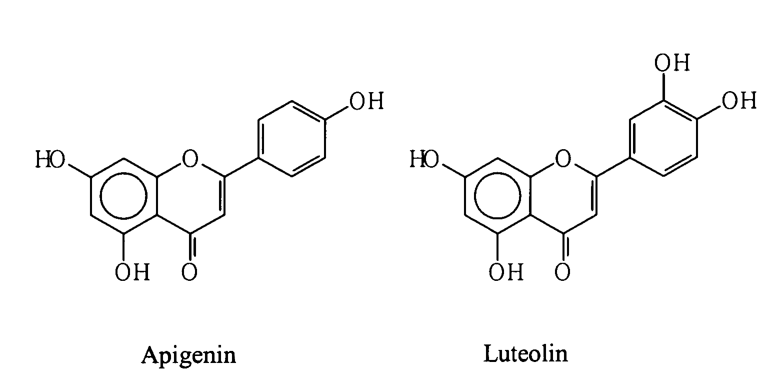

Des weiteren enthalten sie 2"-B-D-Glucosides, Schaftoside and Isoschaftoside, Isovitexin, Isoorientin, Vicenin-2, Incenin-2, Daponanin sowie Spurenelement, nämlich vor allem Kalzium, Phosphor und Eisen.

### • Medicago sativa

Extrakte der Alfalfa (*Medicago sativa*) sind reich an Isoflavonen, wie z.B. Daidzein, Genestein, Formononetin, Biochanin A und Tricin :

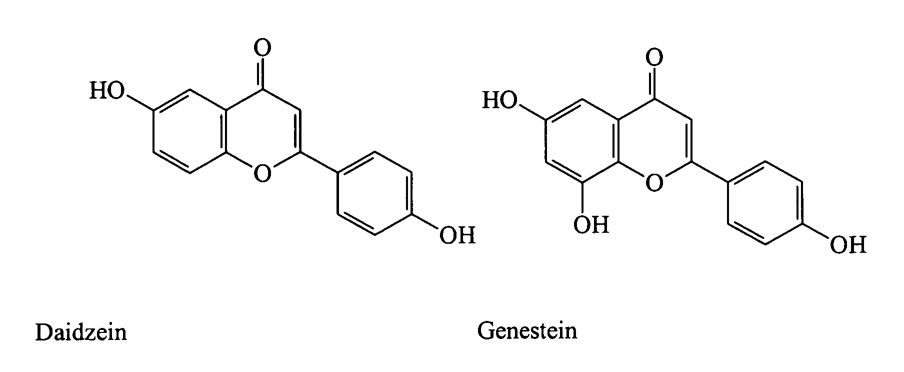

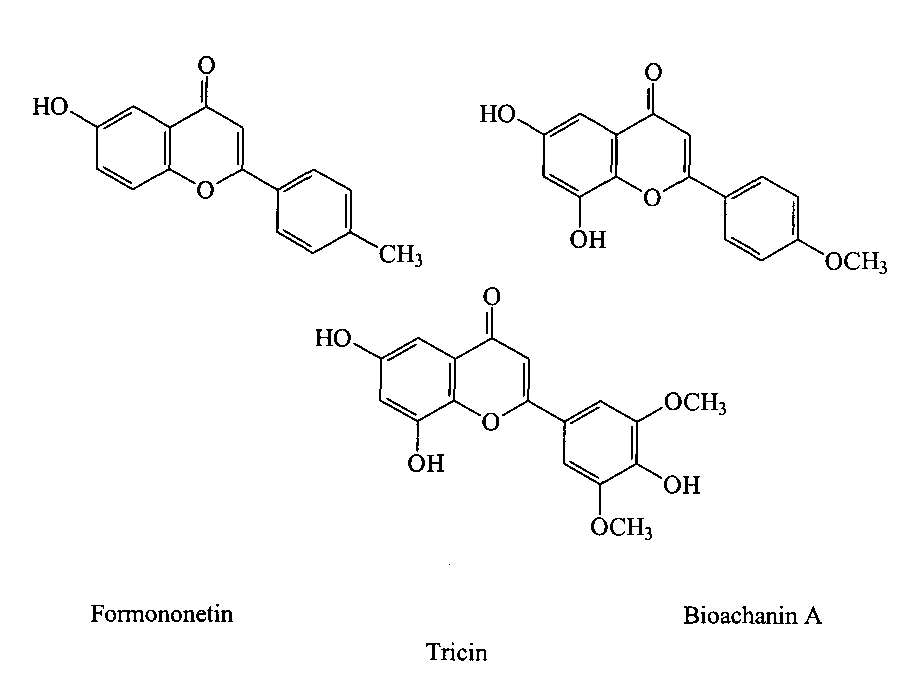

### • Valeriana officinalis

Die Hauptbestandteile von Extrakten der *Valeriana officinalis* sind Valeriansäure, Valerianon sowie Borneolester.

### • Castanea sativa

Rosskastanienextrakte (*Castanea sativa*) enthalten hauptsächlich Saponine sowie Escin, welches die Mischung zweier Glycoside darstellt, deren Aglycone sich von Proteoescigenin ableiten, während es sich bei den Zuckern entweder um Glucoronsäure oder zwei Molekülen D-Glucose handelt. Die beiden Glycoside unterscheiden sich in der Natur der Acylgruppen in der C22-Position.

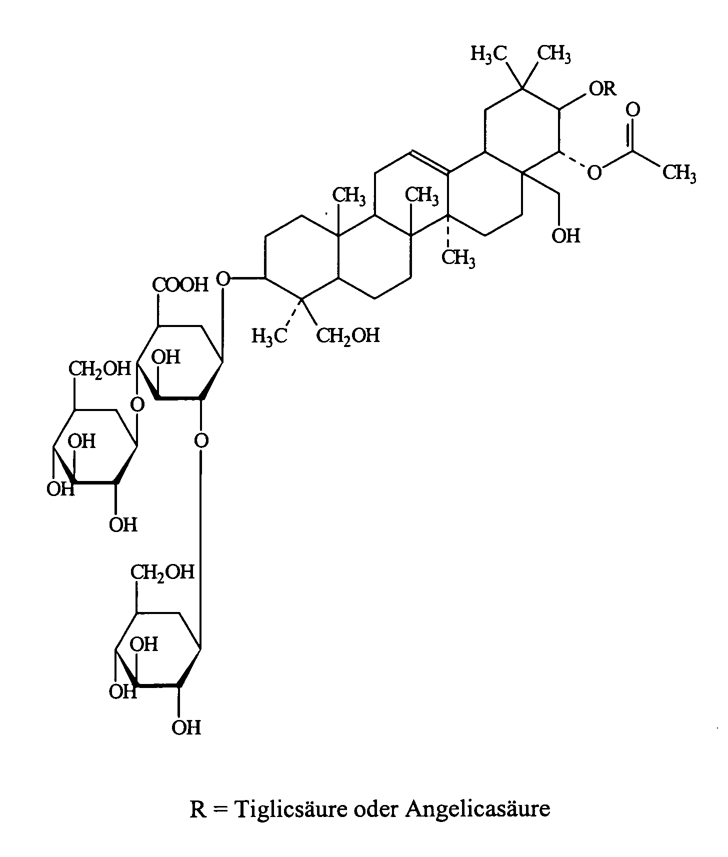

Während α-Escin ein amorphes Pulver darstellt, welches bei 225 bis 227 °C schmilzt und leicht wasserlöslich ist, liegt β-Escin (das auch als Flogencyl bezeichnet wird) in Form von Schuppen vor, die praktisch wasserunlöslich, aber leicht löslich in Alkohol sind.

### • Salix alba

Hauptbestandteile der Extrakte von *Salix alba* sind Phenolglykoside und insbesondere Salicylate wie z.B. Salicin, Salicortin und Tremulacin:

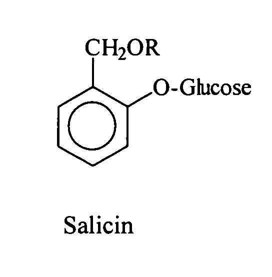

### • Harpagophytum procumbens

Die Hauptbestandteile der Extrakte der Teufelskralle (*Harpagophytum procumbens*) sind Iridoidglucoside, Harpagoside, Harpagide und Procumbide.

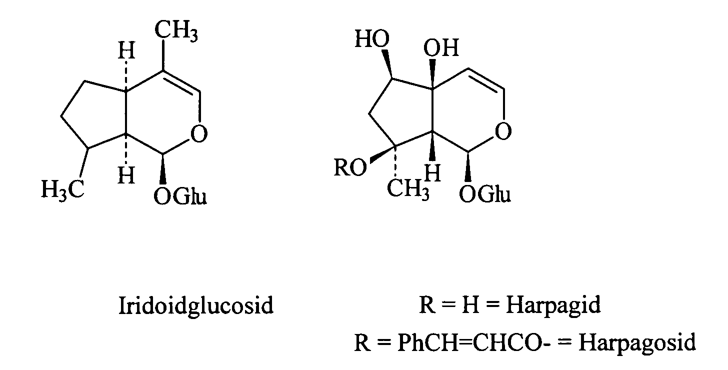

Des weiteren findet man Stachylose und glycosylierte Phytosterole (z.B. β-Sitosterol), Flavonoide (z.B. Kaempferol, Luteolin), Phenolsäuren und glycosidische Phenylpropansäureestem (z.B. Verbacoside, Isoacteoside).

Üblicherweise setzt man die Polymere und die Pflanzenextrakte - jeweils bezogen auf Aktivsubstanz - im Gewichtsverhältnis 10 : 90 bis 90 : 10, vorzugsweise 25 : 75 Bis 75 : 25 und insbesondere 40 : 60 bis 60 : 40 ein.

### Verfestigungsmittel

Um die Beständigkeit der Mikrokapseln im alkalischen Milieu noch weiter zu verbessern hat es sich als vorteilhaft erwiesen, den wässrigen Polymerlösungen als weitere Komponente Verfestigungsmittel zuzusetzen. Für diesen Zweck eignen sich beispielsweise Polyalkylenglykole, insbesondere Polyethylenglykole mit mittleren Molekulargewichten von 200 bis 5.000 und/oder Trialkylcitrate, wie vorzugsweise Triethylcitrat. Bezogen auf den Aktivsubstanzgehalt der Polymerlösungen werden die Verfestigungsmittel vorzugsweise in Mengen von 1 bis 10 und insbesondere 2 bis 5 Gew.-% eingesetzt.

### Herstellung der Mikrokapseln

Zur Herstellung der erfindungsgemäßen Mikrokapseln werden im ersten Schritt wässrige Suspensionen des entsprechenden Polymers vorgelegt und durch Zugabe wässriger Basen auf einen pH-Wert von ca. 8 eingestellt. Hierbei treten die Polymere in Lösung, was in der Regel daran schon optisch leicht zu erkennen ist, dass die milchig-opaken Flüssigkeiten transparent werden. Anschließend wird den Zubereitungen der entsprechende getrocknete Pflanzenextrakt in einer solchen Menge zugesetzt, dass eine Beladung der fertigen Kapseln - bezogen auf Feststoff- bzw. Aktivgehalt - von wenigstens 5 und maximal 90 Gew.-% resultiert. Falls gewünscht, kann den Polymerlösungen auch ein Verfestigungsmittel in Mengen von 1 bis 10 Gew.-% bezogen auf die Polymere zugesetzt werden, um die Kapseln weniger verformbar zu machen. Nach dem Vermischen werden die wässrigen Zubereitungen durch Sprühtrocknung von Wasser befreit.

### Gewerbliche Anwendbarkeit

Ein weiterer Gegenstand der Erfindung betrifft die Verwendung der neuen Mikrokapseln zur Herstellung von Nahrungsmittelergänzungsstoffen oder pharmazeutischen Zubereitungen, in denen sie in Mengen von 0,1 bis 20, vorzugsweise 1 bis 15 und insbesondere 2 bis 10 Gew.-% enthalten sein können.

### Beispiele

### Beispiel 1:

In einer Mischvorrichtung wurden 20 kg einer 30 Gew.-%igen wässrigen Polyacrylat-Suspension (Eudralgit® L30D55, Degussa AG) vorgelegt und mit 40 kg Wasser verdünnt. Die milchige Zubereitung wurde unter starker Durchmischung mit 30 Gew.-%iger Natriumhydroxidlösung auf einen pH-Wert von etwa 8,0 eingestellt, wobei eine transparente Flüssigkeit erhalten wurde. Die Polyacrylatlösung wurde sodann mit 5 kg eines getrocknete Extraktes von *Harpagophytum precumbens* versetzt, wobei eine Mischung mit einem Feststoffgehalt von etwa 11,5 Gew.-% erhalten wurde. Anschließend wurde die Lösung mit Hilfe eines Sprühtrockners von Wasser befreit und es resultierten Mikrokapseln mit einem mittleren Durchmesser von 0,16 mm.

### Beispiel 2:

In einer Mischvorrichtung wurden 20 kg einer 30 Gew.-%igen wässrigen Polyacrylat-Suspension (Eudralgit® L30D55, Degussa AG) vorgelegt und mit 40 kg Wasser verdünnt. Die milchige Zubereitung wurde unter starker Durchmischung mit 30 Gew.-%iger Natriumhydroxidlösung auf einen pH-Wert von etwa 8,0 eingestellt, wobei eine transparente Flüssigkeit erhalten wurde. Die Polyacrylatlösung wurde sodann mit 5 kg eines getrocknete Extraktes von *Salix alba* sowie 3 kg Polyethylenglykol (M = 600) versetzt, wobei eine Mischung mit einem Feststoffgehalt von etwa 11,5 Gew.-% erhalten wurde. Anschließend wurde die Lösung mit Hilfe eines Sprühtrockners von Wasser befreit und es resultierten Mikrokapseln mit einem mittleren Durchmesser von 0,2 mm.

### Beispiel 3:

In einer Mischvorrichtung wurden 20 kg einer 30 Gew.-%igen wässrigen Polyacrylat-Suspension (Eudralgit® L30D55, Degussa AG) vorgelegt und mit 40 kg Wasser verdünnt. Die milchige Zubereitung wurde unter starker Durchmischung mit 30 Gew.-%iger Natriumhydroxidlösung auf einen pH-Wert von etwa 8,0 eingestellt, wobei eine transparente Flüssigkeit erhalten wurde. Die Polyacrylatlösung wurde sodann mit 5 kg eines getrocknete Extraktes von *Ginkgo biloba* sowie 3 kg Triethylcitrat versetzt, wobei eine Mischung mit einem Feststoffgehalt von etwa 11,5 Gew.-% erhalten wurde. Anschließend wurde die Lösung mit Hilfe eines Sprühtrockners von Wasser befreit und es resultierten Mikrokapseln mit einem mittleren Durchmesser von 0,16 mm.

### Beispiel 4:

In einer Mischvorrichtung wurden 20 kg einer 30 Gew.-%igen wässrigen Hydroxypropylmethylcellulosephthalat (HPMCP, Shin-Etsu) vorgelegt und mit 40 kg Wasser verdünnt. Die milchige Zubereitung wurde unter starker Durchmischung mit 30 Gew.-%iger Natriumhydroxidlösung auf einen pH-Wert von etwa 8,0 eingestellt, wobei eine transparente Flüssigkeit erhalten wurde. Die HPMCP-Lösung wurde sodann mit 5 kg eines getrocknete Extraktes von *Camellia sinensis* versetzt, wobei eine Mischung mit einem Feststoffgehalt von etwa 11,5 Gew.-% erhalten wurde. Anschließend wurde die Lösung mit Hilfe eines Sprühtrockners von Wasser befreit und es resultierten Mikrokapseln mit einem mittleren Durchmesser von 0,16 mm.

### Beispiel 5:

In einer Mischvorrichtung wurden 20 kg einer 30 Gew.-%igen wässrigen Hydroxypropylmethylcellulose (AQUOAT, Shin-Etsu) vorgelegt und mit 40 kg Wasser verdünnt. Die milchige Zubereitung wurde unter starker Durchmischung mit 30 Gew.-%iger Natriumhydroxidlösung auf einen pH-Wert von etwa 8,0 eingestellt, wobei eine transparente Flüssigkeit erhalten wurde. Die Polymer-Lösung wurde sodann mit 5 kg eines getrocknete Extraktes von *Valeriana oficinalis* versetzt, wobei eine Mischung mit einem Feststoffgehalt von etwa 11,5 Gew.-% erhalten wurde. Anschließend wurde die Lösung mit Hilfe eines Sprühtrockners von Wasser befreit und es resultierten Mikrokapseln mit einem mittleren Durchmesser von 0,18 mm.

## Patentansprüche

1. Mikrokapseln mit mittleren Durchmessern im Bereich von 0,0001 bis 5 mm, **dadurch** erhältlich, dass man
(a) wässrige Zubereitungen von Polymeren, die bei erst pH-Werten oberhalb von 5,5 löslich sind, auf einen alkalischen pH-Wert einstellt,
(b) die so erhaltenen Lösungen mit Pflanzenextrakten vermischt und
(c) die Zubereitungen einer Sprühtrocknung unterwirft.

2. Verfahren zur Herstellung von Mikrokapseln mit mittleren Durchmessern im Bereich von 0,0001 bis 5 mm, bei dem man'
(a) wässrige Zubereitungen von Polymeren, die erst bei pH-Werten oberhalb von 5,5 löslich sind, auf einen alkalischen pH-Wert einstellt,
(b) die so erhaltenen Lösungen mit Pflanzenextrakten vermischt und
(c) die Zubereitungen einer Sprühtrocknung unterwirft.

3. Verfahren nach Anspruch 2, **dadurch gekennzeichnet, dass** man Polymere einsetzt, die ausgewählt sind aus der Gruppe, die gebildet wird von Polyacrylaten, Polymethacrylaten, Hydroxypropylmethylcellulose und Hydroxypropylmethylcellulosephthalat.

4. Verfahren nach den Ansprüchen 2 und/oder 3, **dadurch gekennzeichnet, dass** man die wässrigen Polymerlösungen auf einen pH-Wert im Bereich von 7,5 bis 11 einstellt.

5. Verfahren nach mindestens einem der Ansprüche 2 bis 4, **dadurch gekennzeichnet, dass** man Pflanzenextrakte einsetzt, die ausgewählt sind aus der Gruppe, die gebildet wird von *Ginkgo biloba, Oleacea europensis, Glyzyrrhiza glabra, Vaccinium myrtillus, Trifolium pratense, Litchi sinensis, Vitis vinifera, Brassica oleracea, Punica granatum, Petroselinium crispum, Centella asiatica, Passiflora incarnata, Medicago sativa, Valeriana officinalis, Castanea sativa, Salix alba* sowie *Hapagophytum procumbens.*

6. Verfahren nach mindestens einem der Ansprüche 2 bis 5, **dadurch gekennzeichnet, dass** man die Polymere und die Pflanzenextrakte - jeweils bezogen auf Aktivsubstanz - im Gewichtsverhältnis 10 : 90 bis 90 : 10 einsetzt.

7. Verfahren nach mindestens einem der Ansprüche 2 bis 6, **dadurch gekennzeichnet, dass** man den wässrigen Polymerlösungen als weitere Komponente Verfestigungsmittel zusetzt.

8. Verfahren nach Anspruch 7, **dadurch gekennzeichnet, dass** man als Verfestigungsmittel Polyalkylenglykole und/oder Trialkylcitrate einsetzt.

9. Verfahren nach den Ansprüchen 7 und/oder 8, **dadurch gekennzeichnet, dass** man bezogen auf den Aktivsubstanzgehalt der Polymerlösungen 1 bis 10 Gew.-% Verfestigungsmittel einsetzt.

10. Verwendung von Mikrokapseln nach Anspruch 1 zur Herstellung von Nahrungsmittelergänzungsstoffen oder pharmazeutischen Zubereitungen.
